# EUROPEAN PATENT SPECIFICATION

(11) **EP 1 383 508 B1**
(45) Date of publication and mention of the grant of the patent: **21.06.2006**
(21) Application number: 02731267.7
(22) Date of filing: 05.04.2002
(51) Int. Cl.: A61K 31/65, A61P 17/00, A61P 17/10, A61P 43/00

(54) **DOXYCYCLINE FOR THE TREATMENT OF ACNE**
DOXYCYCLIN ZUR BEHANDLUNG VON AKNE
DOXYCYCLINE DESTINEE AU TRAITEMENT DE L'ACNE

(30) Priority: 05.04.2001 US 281916 P
(43) Date of publication of application: 28.01.2004
(73) Proprietor: Collagenex Pharmaceuticals, Inc., Newtown, PA 18940 (US)
(72) Inventor: ASHLEY, Robert, A., Newtown, PA 18940 (US)
(74) Representative: Prins, Adrianus Willem
(86) International application number: PCT/US2002/010747
(87) International publication number: WO 2002/080932

(56) References cited:
- EP-A- 0 410 099
- WO-A-83/00628
- US-A- 5 674 539
- US-A- 5 827 840
- US-A- 5 908 838
- SKIDMORE ROBERT ET AL: "Effects of subantimicrobial-dose doxycycline in the treatment of moderate acne." ARCHIVES OF DERMATOLOGY. APR 2003, vol. 139, no. 4, April 2003 (2003-04), pages 459-464, XP009047590 ISSN: 0003-987X

## Description

### BACKGROUND OF THE INVENTION

Acne is a common disease characterized by various types of lesions. The areas affected typically are areas of the skin where sebaceous glands are largest, most numerous, and most active. The lesions associated with acne are usually categorized as either non-inflammatory or inflammatory.

Non-inflammatory lesions include comedones. Comedones appear in two forms, open and closed. Comedones are thought to arise from abnormal follicular differentiation. Instead of undergoing shedding and discharge through the follicular orifice, abnormal desquamated cells (keratinocytes) become unusually cohesive, forming a microcomedo or a microscopic hyperkeratotic plug in the follicular canal. The progressive accumulation of these microcomedones lead to visible comedones.

In its mildest form, acne is a more or less superficial disorder characterized by slight, spotty skin irritations. In such cases, ordinary skin hygiene is typically a satisfactory treatment. In the more inflammatory types of acne, however, pustules; infected cysts; and in extreme cases, canalizing, inflamed and infected sacs appear. Without effective treatment, these lesions may become extensive and leave permanent, disfiguring scars.

Microorganisms, *especially Propionibacterium acnes,* are strongly implicated in the pathogenesis of acne. The microorganisms are thought to release microbial mediators of inflammation into the dermis or trigger the release of cytokines from ductal keratinocytes.

Accordingly, the efficacy of antibiotics in treating acne is thought to be due, in significant part, to the direct inhibitory effect of the antibiotics on the growth and metabolism of these microorganisms. Systemically-administered tetracycline antibiotics, especially minocycline hydrochloride, are particularly effective in treating acne.

The tetracyclines are a class of compounds of which tetracycline is the parent compound. Tetracycline has the following general structure: 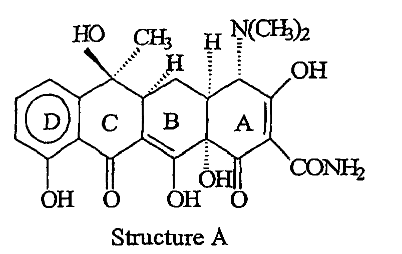 The numbering system of the multiple ring nucleus is as follows:

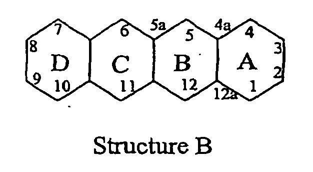

Tetracycline, as well as the 5-hydroxy (oxytetracycline, e.g. Terramycin) and 7-chloro (chlorotetracycline, e.g. Aureomycin) derivatives, exist in nature, and are all well known antibiotics. Semisynthetic derivatives such as 7-dimethylaminotetracycline (minocycline) and 6a-deoxy-5-hydroxytetracycline (doxycycline) are also known tetracycline antibiotics. Natural tetracyclines may be modified without losing their antibiotic properties, although certain elements of the structure must be retained to do so.

In addition to the direct antibiotic activity of tetracyclines, further activities of antibiotic tetracyclines have been investigated for possible therapeutic effects on acne.

For example, a study by Elewski et al., *J. Amer. Acad. Dermatol., 8*:807-812 (1983) suggests that acne therapy, consisting of orally-administered tetracycline at a total daily dose of 1000 mg, may have therapeutic anti-inflammatory effects in addition to antibiotic effects. In particular, it was found that the anti-inflammatory effect of tetracycline was, at least in part, due to inhibition of neutrophil chemotaxis induced by bacterial chemotactic factors.

A more recent study, performed by Eady et al., *J. Invest. Dermatol., 101:* 86-91 (1993), evaluated the effects of oral minocycline or tetracycline therapy on the cytokine and microflora content of open comedones in acne patients. The total daily dose of minocycline administered was 100 mg. The total daily dose of tetracycline administered was 1000 mg.

Eady et al. found that the therapies upregulated the production of bioactive IL-1α-like material and immunochemical IL-1β. IL-1 is considered to be a pro-inflammatory cytokine.

Accordingly to Eady et al., no overall decrease in the numbers of propionibacteria/mg of comedonal material was found. It is important to note, however, that the numbers of propionibacteria/mg of comedonal material are not expected to decrease in response to antibiotic therapy. Since the bacteria within comedones are encapsulated by the follicle, they are not susceptible to antibiotic treatment.

Another possible activity of tetracyclines in acne therapy was investigated by Bodokh, I., et al., *Acta. Derm. Venerol.,* 77:255-259 (1997). Their study was designed to evaluate the action of minocycline on sebaceous excretion in acne patients. A 100 mg daily dose of minocycline was administered. A subclinical increase in seborrhoea was reported. The authors propose that minocycline induces an increase in seborrhoea via a reduction in ductal obstruction. The mechanism by which the ductal obstruction is reduced is proposed to be a reduction in ductal irritation. The authors suggest that the reduction of ductal irritation is due to minocycline's direct effect on *P*. *acnes,* or minocycline's effect on the lipase produced by *P. acnes.*

Bodokh et al. also found that during treatment no correlation exists between seborrhoea intensity and clinical severity of acne. The authors state that the lack of correlation shows that seborrhoea is pathogenic because it is the "culture medium" of *P. acnes.* Thus, it can be concluded that the authors consider the antibiotic activity of minocycline to be therapeutically significant with respect to acne.

Similarly, in a recent clinical study it was reported that tetracycline in sub-antibiotic doses had no clinical effect on acne. (Cunliffe et al., *J*. *Am. Acad. Dermatol.,16:*591-9 (1987).) In particular, a 100 mg total daily dose of minocycline and a 1.0g total daily dose of tetracycline were found to be necessary to successfully treat acne.

The antibiotic effects of antibiotics are generally directly proportional to the dose administered of the antibiotics. Accordingly, in moderate to severe (i.e. inflammatory) forms of acne, oral antibiotics are typically administered at high doses. For example, in conventional acne therapy, tetracycline is administered at an initial dose of 500 to 2,000 mg/day, followed by a maintenance dose of 250-500 mg/day.

Clearly, the state-of-the-art teaching is that the clinical efficacy of systemically-administered tetracyclines in the treatment of acne is due, at least in significant part, to the antibiotic effects of the tetracyclines. In addition to their antibiotic effects, it has been proposed that tetracyclines reduce the number of inflammatory lesions (papules, pustules and nodules) by a variety of non-antibiotic mechanisms. Such mechanisms include interfering with the chemotaxis of polymorphonuclear leukocytes (PMN) into the inflammatory lesion, inhibition of PMN derived collagenase, and by scavenging reactive oxidative species produced by resident inflammatory cells.

US patent 5,908,838 discloses a method for reducing the incidence or severity of vestibular side effects resulting from the treatment of acne by the use of oral tetracycline antibiotics, comprising administering the oral tetracycline antibiotic in a slowly dissolving form.

US patent 5,674,539 discloses a topical medication for the treatment of skin pathology including acne comprising tetracycline, minocycline, clindamycin, erythromycin, or doxicyclin and selenium sulphide or sulphur in a pharmaceutically acceptable carrier. It is mentioned that, because the treatment is local, the serious side effects of systemic administration are avoided.

European patent application 0 410 099 discloses topical gel formulation of tetracycline antibiotics for the topical treatment of acne in humans.

WO 83/00628 discloses a method of treating acne vulgaris comprising administering a therapeutically effective amount of carbamide peroxide alone or in combination with one or more of a topical antibiotic, nicotinic acid or nicotinamide and compositions useful in said method.

There is no disclosure in the prior art of using either a sub-antibiotic dose of an antibiotic tetracycline compound, or of using a non-antibiotic tetracycline compound for the treatment of acne.

The use of tetracycline antibiotics, however, can lead to undesirable side effects. For example, the long term administration of antibiotic tetracyclines can reduce or eliminate healthy microbial flora, such as intestinal flora, and can lead to the production of antibiotic resistant organisms or the overgrowth of yeast and fungi.

Accordingly, there is a need for an effective treatment of acne which causes fewer undesirable side effects produced by the systemically-administered antibiotics used in conventional acne therapy.

### SUMMARY OF INVENTION

The present invention provides a use of a tetracycline compound for the manufacture of a medicament for treating acne in a human in need thereof as defined in claim 1. The medicament is to be administered systemically to the human in an amount that is effective to treat acne but has substantially no antibiotic activity (i.e. substantially no antimicrobial activity), without administering a bisphosphonate compound.

Additionally, the medicament is useful for reducing the number of comedones, inhibiting oxidation of melanin, and/or inhibiting lipid-associated abnormal follicular differentiation in a human in need thereof These methods comprise administering systemically to the human a tetracycline compound in an amount that is effective for its purpose, e.g., to reduce the number of comedones, to inhibit oxidation of melanin, and/or to inhibit lipid-associated abnormal follicular differentiation, but has substantially no antibiotic activity.

### BRIEF DESCRIPTION OF THE DRAWINGS

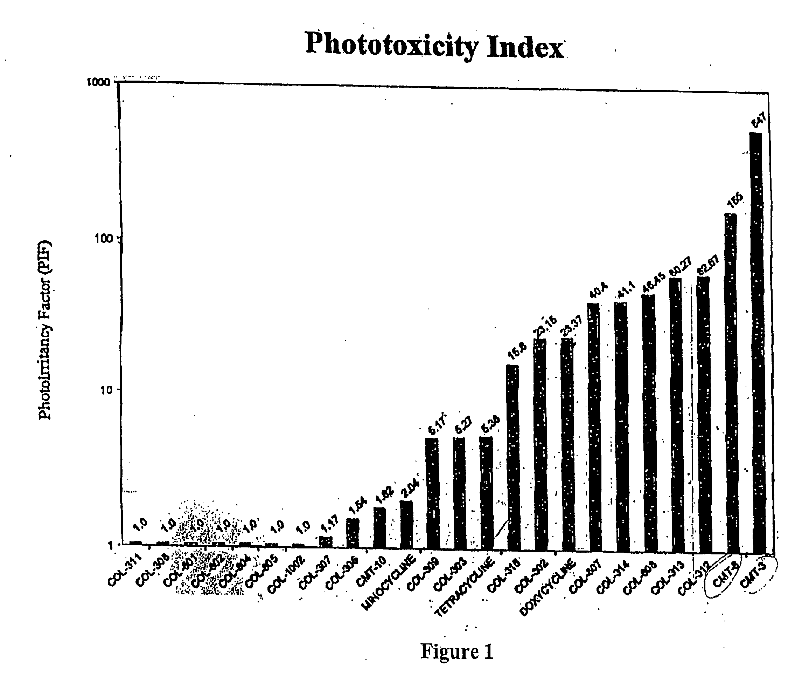

Figure 1 shows the photoirritancy factor (PIF) for some tetracycline compounds. For structure K, the compounds indicated are as follows:

| COL | R7 | R8 | R9 |
|---|---|---|---|
| 308 | hydrogen | hydrogen | amino |
| 311 | hydrogen | hydrogen | pabnitamaide |
| 306 | hydrogen | hydrogen | dimethylamino |

For structures L, M, N or O the compounds indicated are as follows:

| | | | |
|---|---|---|---|
| COL | R7 | R8 | R9 |
| 801 | hydrogen | hydrogen | acetamido |
| 802 | hydrogen | hydrogen | dimethylaminoacetamido |
| 804 | hydrogen | hydrogen | nitro |
| 805 | hydrogen | hydrogen | amino |

For structure P, R8 is hydrogen and R9 is nitro.

### DETAILED DESCRIPTION

As used herein; the term "acne" is a disorder of the skin characterized by papules, pustules, cysts, nodules, comedones, and other blemishes or skin lesions. These blemishes and lesions are often accompanied by inflammation of the skin glands and pilosebaceous follicles, as well as, microbial, especially bacterial, infection.

For the purposes of this specification, acne includes all known types of acne. Some types of acne include, for example, acne vulgaris, cystic acne, acne atrophica, bromide acne, chlorine acne, acne conglobata, acne cosmetica, acne detergicans, epidemic acne, acne estivalis, acne fulminans, halogen acne, acne indurata, iodide acne, acne keloid, acne mechanica, acne papulosa, pomade acne, premenstral acne, acne pustulosa, acne scorbutica, acne scrofulosorum, acne urticata, acne varioliformis, acne venenata, propionic acne, acne excoriee, gram negative acne, steroid acne, nodulocystic acne and acne rosacea. Acne rosacea is characterized by inflammatory lesions (erythema) and permanent dilation of blood vessels (telangectasia).

The present medicament is particularly effective in treating comedones, e.g., reducing the number of comedones. Both open and closed comedones can be treated using the medicament of this invention.

The present invention can also be used to treat certain other types of acneiform dermal disorders, e.g. perioral dermatitis, seborrheic dermatitis in the presence of acne, gram negative folliculitis, sebaceous gland dysfunction, hiddradenitis suppurativa, pseudo-folliculitis barbae, or folliculitis.

In accordance with the invention, tretacycline compound is doxycycline, or a pharmaceutically acceptable salt therof Doxycycline is preferably administered as its hyclate salt or as a hydrate, preferably monohydrate.

The minimal amount of doxycycline administered to a human is the lowest amount capable of providing effective treatment of acne. Effective treatment is a reduction or inhibition of the blemishes and lesions associated with acne. The amount of the tetracycline compound is such that it does not significantly prevent the growth of microbes, e.g. bacteria.

Two ways in which to describe the administered amount of doxycycline is by daily dose, and by serum level.

For example, doxycycline may be administered in a dose (i.e. amount) which is 10-80% of the antibiotic dose. More preferably, the doxycycline is administered in a dose which is 40-70% of the antibiotic dose.

Some examples of antibiotic doses of doxycycline include 50,75, and 100 mg/day.

Examples of the maximum non-antibiotic doses on steady-state pharmacokinetics are as follows: 20 mg/twice a day for doxycycline,

In a preferred embodiment, to reduce the number of comedones, doxycycline is administered in a daily amount of from about 30 to about 60 milligrams, but maintains a concentration in human plasma below the threshold for a significant antibiotic effect.

In an especially preferred embodiment, doxycycline hyclate is administered at a 20 milligram dose twice daily. Such a formulation is sold for the treatment of periodontal disease by CollaGenex Pharmaceuticals, Inc. of Newtown, Pennsylvania under the trademark Periostat ®.

Example 1 below summarizes a clinical study using 20 mg doxycycline hyclate tablets administered twice a day. A significant reduction in the number of comedones was observed. This reduction in the number of comedones is unexpected. The reduction is particularly unexpected since, as can be seen from the microbiology results in Example 1, the treatment with doxycycline resulted in no reduction of skin microflora vis-à-vis a placebo control.

The adminstered amount of doxycycline described by serum levels follows.

Doxycycline is advantageously administered in an amount that results in a serum tetracycline concentration which is 10-80% of the minimum antibiotic serum concentration. The minimum antibiotic serum concentration is the lowest concentration known to exert a significant antibiotic effect.

In one embodiment, the doxycycline can be administered in an amount which results in a serum concentration between about 0.1 and 10.0 µg/ml, more preferably between 0.3 and 5.0 µg/ml. For example, doxycycline is administered in an amount which results in a serum concentration between about 0.1 and 0.8 µg/ml, more preferably between 0.4 and 0.7 µg/ml.

One examples the plasma antibiotic threshold levels based on steady-state pharmacokinetics is 1.0 *µ*g/ml for doxycycline.

The actual preferred amounts of doxycycline in a specified case will vary according to the particular compositions formulated, the mode of application, the particular sites of application, and the subject being treated.

The doxycycline can be in the form of pharmaceutically acceptable salts of the compounds. The term "pharmaceutically acceptable salt" refers to a salt prepared from tetracycline compounds and pharmaceutically acceptable non-toxic acids or bases. The acids may be inorganic or organic acids of tetracycline compounds. Examples of inorganic acids include hydrochloric, hydrobromic, hydroiodic, sulfuric, and phosphoric acids. Examples of organic acids include carboxylic and sulfonic acids. The radical of the organic acids may be aliphatic or aromatic. Some examples of organic acids include formic, acetic, phenylacetic, propionic, succinic, glycolic, glucuronic, maleic, furoic, glutamic, benzoic, anthranilic, salicylic, phenylacetic, mandelic, embonic (pamoic), methanesulfonic, ethanesulfonic, panthenoic, benzenesulfonic, stearic, sulfanilic, alginic, tartaric, citric, gluconic, gulonic, arylsulfonic, and galacturonic acids. Appropriate organic bases may be selected, for example, from N,N-dibenzylethylenediamine, chlomprocaine, choline, diethanolamine, ethylenediamine, meglumine (N-methylglucamine), and procaine.

Doxycycline, is unexpectedly effective in reducing the number of comedones when administered at a dose which has substantially no antibiotic effect. Preferably the reduction is at least about 20% greater than for a placebo control, more preferably at least about 30% greater than for a placebo control, most preferably at least about 40% greater than for a placebo control, and optimally at least about 50% greater than for a placebo control.

The inventors are not certain of, and do not wish to be limited by, any particular mechanism of action. Nevertheless, it is believed that the ability of tetracyclines, such as doxycycline, to inhibit oxidation of melanin and to inhibit lipid-associated abnormal follicular differentiation prevents keratinocytes from becoming cohesive, thereby inhibiting the formation of comedones.

Preferably, the tetracycline compound has low phototoxicity, or is administered in an amount that results in a serum level at which the phototoxicity is acceptable. Phototoxicity is a chemically-induced photosensitivity. Such photosensitivity renders skin susceptible to damage, e.g. sunburn, blisters, accelerated aging, erythemas and eczematoid lesions, upon exposure to lights in particular ultraviolet light The preferred amount of doxycycline produces no more phototoxicity than is produced by the administration of a 40 mg total daily dose of doxycycline.

Phototoxicity can be evaluated in terms of a photoirritancy factor (PIF), as described in the examples. A PIF value of about 1.0 indicates that a compound is considered to have no measurable phototoxicity.

Doxycycline is to be administered without administering a bisphosphonate compound. Bisphosphonates compounds are related to inorganic pyrophosphonic acid. The bisphosphonates include alendronate ((4-amino-1- hydroxybutylidene) bisphosphonic acid), clodronate (dichloromethane diphosphonic acid), etidronate ((1-hydroxyethylidene) diphosphanic acid) and pamidronate ((3-amino-1- hydroxypropylidene) bisphosphonic acid); also risedronate ([-hydroxy-2-(3-pyridinyl)ethylidene] bisphosphonic acid), tiludronate, i.e., tiludronic acid ([(4-chlorophenyl) thio] methylene] bisphosphonic acid) and zolendronate.

Doxycycline is to be administered systemically. For the purposes of this specification, "systemic administration" means administration to a human by a method that causes the compounds to be absorbed into the bloodstream.

For example, doxycyline can be administered orally by any method known in the art For example, oral administration can be by tablets, capsules, pills, troches, elixirs, suspensions, syrups, wafers, and chewing gum.

Additionally, doxycycline can be administered enterally or parenterally, e.g., intravenously; intramuscularly; subcutaneously, as injectable solutions or suspensions; intraperitoneally; or rectally. Administration can also be intranasally, in the form of, for example, an intranasal spray

For the pharmaceutical purposes described above, doxycycline can be formulated per se in pharmaceutical preparations optionally with a suitable pharmaceutical carrier (vehicle) or excipient as understood by practitioners in the art. These preparations can be made according to conventional chemical methods.

In the case of tablets for oral use, carriers which are commonly used include lactose and com starch, and lubricating agents such as magnesium stearste are commonly added. For oral administration in capsule form, useful carriers include lactose and corn starch. Further examples of carriers and excipients include milk, sugar, certain types of clay, gelatin, stearic acid or salts thereof, calcium stearate, talc, vegetable fats or oils, gums and glycols.

When aqueous suspensions are used for oral administration, emulsifying and/or suspending agents are commonly added. In addition, sweetening and/or flavoring agents may be added to the oral compositions.

For intramuscular, intraperitoneal, subcutaneous and intravenous use, sterile solutions of the tetracycline compounds can be employed, and the pH of the solutions can be suitably adjusted and buffered. For intravenous use, the total concentration of the solute(s) can be controlled in order to render the preparation isotonic.

The medicaments of the present invention can further comprise one or more pharmaceutically acceptable additional ingredient(s) such as alum, stabilizers, buffers, coloring agents, flavoring agents, and the like.

Doxycycline may be administered intermittently. For example, 1-6 times a day, preferably 1-4 times a day.

Alternatively, the doxycycline may be administered by sustained release. Sustained release administration is a method of drug delivery to achieve a certain level of the drug over a particular period of time. The level typically is measured by serum concentration. Further description of methods of delivering tetracycline compounds by sustained release can be found in the patent application, "Controlled Delivery of Tetracycline and Tetracycline Derivatives," filed on April 5, 2001 and assigned to CollaGenex Pharmaceuticals, Inc. of Newtown, Pennsylvania. For example, 40 milligrams of doxycycline may be administered by sustained release over a 24 hour period.

### EXAMPLES

### EXAMPLE 1

### Effects of Doxycycline Hyclate 20 mg (Dermastat) Tablets Administered Twice Daily for the Treatment of Moderate Acne

### Study Design:

- Multi-center, randomized double-blind, placebo-controlled;
- Sixty patients enrolled (30 doxycycline and 30 placebo);
- Six month duration of the study.
- Patients received medication twice daily, approximately 12 hours apart (placebo and drug are identical in appearance.)

### Inclusion Criteria:

- Healthy post-pubescent males and females (age ≥ 18) with moderate facial acne:
   - Comedones 6 to 200;
   - Inflammatory lesions 10 to 75 (papules and pustules, less than or equal to 5 nodules);
- Females tested negative for pregnancy and were non-lactating;
- Females maintained appropriate birth control;
- Patients signed an Informed Consent Form;
- No Accutane treatment for 6 months prior to baseline.

### Exclusion Criteria:

- Use of hormonal contraception 6 months prior to baseline or during study;
- Use of topical acne treatments within 6 weeks of baseline or during study;
- Use of systemic antibiotics within 6 weeks of baseline or during study;
- Use of investigational drugs within 90 days of baseline;
- Use of any acne treatments during study.

### Study Procedure:

- Patients reported to clinician at baseline and months 2, 4, and 6;
- Acne counts were taken at baseline and months 2, 4, and 6;
- Patient self-assessment and clinician's assessment (baseline, 2, 4, and 6);
- Facial photographs at baseline and months 2, 4, and 6;
- Drug dispensation at baseline and months 2 and 4;
- Adverse event recording at baseline and months 2, 4, and 6;
- Microbiological sampling at baseline and month 6;
- Clinical Labs at baseline and month 6.

### Evaluations:

### Efficacy:

- Change in lesion count of papules and pustules
- Change in comedone count
- Change in total lesion count (comedones and inflammatory lesions)

### Microbiology:

- Reduction in skin flora between groups
- Increase in resistant counts between groups

### Efficacy Results

A six-month treatment with Dermastat resulted in: *i*) a 53.6% reduction in comedones vis-à-vis a 10.6% reduction of comedones in placebo (p<0.05); *ii*) a 50.1% reduction in inflammatory lesions vis-à-vis a 30.2% reduction of inflammatory lesions in placebo (p<0.01); and *iii*) a 52.3% reduction in total lesion count vis-à-vis a 17.5% reduction of inflammatory lesions in placebo (p<0.05).

### Microbiology Results:

A six-month treatment with Dermastat resulted in no reduction of skin microflora (including *Propionibacterium acnes)* nor an increase in resistance counts when compared with placebo.

## Claims

1. Use of a tetracycline compound for the manufacture of a medicament for treating acne in a human in need thereof, wherein the tetracycline compound is doxycycline or a pharmaceutically acceptable salt thereof and is to be administered systemically to said human in an amount that is effective to treat acne but has no antibiotic activity, without administering a bisphosphonate compound

2. Use according to Claim 1, wherein said acne is acne vulgaris, cystic acne, acne atrophica, bromide acne, chlorine acne, acne conglobata, acne cosmetica, acne detergicans, epidemic acne, acne estivalis, acne fulminans, halogen acne, acne indurata, iodide acne, acne keloid, acne mechanica, acne papulosa, pomade acne, premenstral acne, acne pustulosa, acne rosacea, acne scorbutica, acne scrofulosorum, acne urticata, acne varioliformis, acne venenata, propionic acne, acne excoriee, gram negative acne, steroid acne, or nodulocystic acne.

3. Use according to Claim 1, wherein said tetracycline compound is to be administered in an amount which is 10-80% of the antibiotic amount.

4. Use according to Claim 1, wherein said tetracycline compound is to be administered twice a day in a dose of 20 mg.

5. Use according to Claim 1, wherein said tetracycline compound is to be administered in an amount which results in a serum concentration which is 1.0 µg/ml.

6. Use according to Claim 1, wherein said tetracycline compound is to be administered in an amount which provides a serum concentration in the range of about 0.1 to about 0.8 µg/ml.

7. Use according to Claim 1, wherein said tetracycline compound is to be administered by sustained release over a 24 hour period.

8. Use according to Claim 7, where said tetracycline compound is to be administered in an amount of 40 milligrams.

9. Use according to Claim 1, wherein said systemic administration is oral administration, intravenous injection, intramuscular injection, subcutaneous administration, transdermal administration or intranasal administration.

## Patentansprüche

1. Verwendung einer Tetracyclinverbindung zur Herstellung eines Medikaments zur Behandlung von Akne bei einem Menschen, der eine solche Behandlung benötigt, wobei es sich bei der Tetracyclinverbindung um Doxycyclin oder ein pharmazeutisch annehmbares Salz davon handelt, das an den Menschen systemisch in einer Menge, die bezüglich der Behandlung von Akne effektiv ist, aber keine antibiotische Wirkung hat, verabreicht wird, ohne eine Bisphosphonatverbindung zu verabreichen.

2. Verwendung gemäß Anspruch 1, wobei es sich bei der Akne um Acne vulgaris, Zystenakne, Acne atrophica, Brom-Akne, Chlor-Akne, Acne conglobata, Acne cosmetica, Acne detergicans, epidemische Akne, Acne estivalis, Acne fulminans, Halogenakne, Acne indurata, Iodakne, Keloid-Akne, Acne mechanica, Acne papulosa, Pomade-Akne, prämenstruelle Akne, Acne pustulosa, Acne rosacea, Acne scorbutica, Acne scrofulosorum, Acne urticata, Acne varioliformis, Acne venenata, Acne propionica, Acne excoriee, Gram-negative Akne, Steroid-Akne oder Acne nodulocystica handelt.

3. Verwendung gemäß Anspruch 1, wobei die "fetracyclinverbindung in einer Menge zu verabreichen ist, die 10-80% der antibiotischen Menge entspricht.

4. Verwendung gemäß Anspruch 1, wobei die Tetracyclinverbindung zweimal am Tag in einer Dosis von 20 mg zu verabreichen ist.

5. Verwendung gemäß Anspruch 1, wobei die Tetracyclinverbindung in einer Menge zu verabreichen ist, die zu einer Serumkonzentration führt, die 1,0 µg/ml beträgt.

6. Verwendung gemäß Anspruch 1, wobei die Tetracyclinverbindung in einer Menge zu verabreichen ist, die eine Serumkonzentration im Bereich von etwa 0,1 bis etwa 0,8 µg/ml ergibt.

7. Verwendung gemäß Anspruch 1, wobei die Tetracyclinverbindung durch nachhaltige Freisetzung über einen Zeitraum von 24 Stunden zu verabreichen ist.

8. Verwendung gemäß Anspruch 7, wobei die Tetracyclinverbindung in einer Menge von 40 Milligramm zu verabreichen ist.

9. Verwendung gemäß Anspruch 1, wobei es sich bei der systemischen Verabreichung um orale Verabreichung, intravenöse Verabreichung, intramuskuläre Verabreichung, subkutane Verabreichung, transdermale Verabreichung oder intranasale Verabreichung handelt.

## Revendications

1. Utilisation d'un composé de tétracycline pour la fabrication d'un médicament destiné au traitement de l'acné chez un être humain en ayant besoin, dans laquelle le composé de tétracycline est la doxycycline ou un de ses sels pharmaceutiquement acceptables et doit être administré par voie systémique audit être humain en une quantité qui est efficace pour le traitement de l'acné, mais qui n'a pas d'activité antibiotique, sans administration d'un composé bisphosphonate.

2. Utilisation selon la revendication 1, dans laquelle ladite acné est l'acné vulgaire, l'acné kystique, l'acné atrophique, l'acné bromique, l'acné chlorique, l'acné conglobata, l'acné cosmétique, l'acné detergicans, l'acné épidémique, l'acné estivale, l'acné fulminante, l'acné halogénique, l'acné indurée, l'acné iodique, l'acné chélordienne, l'acné mécanique, l'acné papuleuse, l'acné de pommade, l'acné prémenstruelle, l'acné pustuleuse, l'acné rosacée, l'acné scorbutique, l'acné scrofulosorum, l'acné urticante, l'acné varioliforme, l'acné de contact, l'acné propionique, l'acné excoriée, l'acné gram-négative, l'acné stéroïdienne ou l'acné nodulokystique.

3. Utilisation selon la revendication 1, dans laquelle ledit composé de tétracycline doit être administré en une quantité qui est de 10-80 % de la quantité antibiotique.

4. Utilisation selon la revendication 1, dans laquelle ledit composé de tétracycline doit être administré deux fois par jour en une dose de 20 mg.

5. Utilisation selon la revendication 1, dans laquelle ledit composé de tétracycline doit être administré en une quantité entraînant une concentration sérique qui est de 1,0 µg/ml.

6. Utilisation selon la revendication 1, dans laquelle ledit composé de tetracycline doit être administré en une quantité qui donne une concentration sérique se situant dans la plage d'environ 0, 1 à environ 0,8 µg/ml.

7. Utilisation selon la revendication 1, dans laquelle ledit composé de tétracycline doit être administré par libération prolongée sur une période de 24 heures.

8. Utilisation selon la revendication 7, dans laquelle ledit composé de tétracycline doit être administré en une quantité de 40 milligrammes.

9. Utilisation selon la revendication 1, dans laquelle ladite administration systémique est une administration orale, une injection intraveineuse, une injection intramusculaire, une administration sous-cutanée, une administration transdermique ou une administration intranasale.
